# EUROPEAN PATENT APPLICATION

(11) **EP 3 231 475 A1**
(43) Date of publication of application: **18.10.2017**
(21) Application number: 17162333.3
(22) Date of filing: 22.03.2017
(51) Int. Cl.: A61N 1/04, A61N 1/32, A61N 1/36

(54) **PROCESS FOR ESTABLISHING AN ELECTROSTIMULATION PROTOCOL FOR PELVIC PAIN CONTROL, AND THE RESPECTIVE PORTABLE ELECTROSTIMULATION EQUIPMENT FOR PELVIC PAIN CONTROL USING SAID PROTOCOL**

(30) Priority: 31.03.2016 BR 102016007241
(71) Applicant: Medecell S.A., Montevideo (UY)
(72) Inventor: Marques de Oliveira, Mauricio, São Paulo (BR); Bighetti, Moacyr Ramos, São Paulo (BR)
(74) Representative: Michalski Hüttermann & Partner Patentanwälte mbB

(57) **Abstract**

These electrostimulation processes and equipment are used in the control of pelvic pain of different etiologies, more particularly, pain resulting from dysmenorrhea and endometriosis, the process predicting the development of an electrostimulation protocol in which the variation of the intensity of the electric pulses is performed in a random manner, respecting the limits of efficacy of the stimulation, in order to reduce the physiological phenomenon of nerve fiber accommodation upon stimuli; and a portable electrostimulation device, consisting of a double bandage (1) formed by two sheets (2), each comprised of central portion (2a) and two side flaps (2b), the central portion (2a) of both sheets (2) housing between them a compartment (3), housing for the electronic module of the apparatus, and the two side flaps (2a) of the top sheet (2) housing the respective electrodes (4), duly covered by the respective gel layers protected by removable protective sheets (5); each one of the sheets (2) is additionally endowed with a respective fastening strip (6); a coin-shaped lithium-ion supply battery (8), model CR20XX, a single on-off button (9), and the following internal components are provided: Power source module (10), step-up regulator module (11), micro controller module (12), power supply seal module (13), boost source module (14), H-bridge module (15), and electrode output module (16).

## Description

### TECHNICAL FIELD

As known in the state of the art, Transcutaneous Electrical Nerve Stimulation (TENS) is a well-known and already consecrated modality of non-medicament and non-invasive treatment for pain control in several etiologies. Such treatment consists in the placement of electrodes in determined regions of the human body, and in the application of electric pulses with the purpose of stimulating the nerves fibers (or nerves); this electrical stimulation produces an analgesic effect generating a reduction or even total elimination of the pain.

This treatment mode has already been used in several clinical scenarios for the treatment of several acute and chronic pain conditions, and have been well accepted among health professionals.

TENS is particularly indicated in cases of painful affections of the most varied etiologies, both acute and chronic. Even visceral pains, such as pelvic pain (caused by dysmenorrhea) and pain of oncological origin, have also been successfully treated by electrostimulation. It is an alternative or an adjuvant to the medicated analgesic treatment, also reducing the need for anti-inflammatories.

More specifically, as for pelvic pain, it is well known the fact that dysmenorrhea is an affection that affects 50% of women of childbearing age.. Many of these women become incapacitated for work activity, as this syndrome causes severe abdominal pain, headache, nausea and even fainting. Thus, millions of work hours are lost because of dysmenorrhea, and treatment is usually carried out based on anti-inflammatories and analgesics, drugs that have several side effects. Given the severity of a significant portion of cases, treatment is often performed in clinics and hospitals.

As an alternative, for the treatment of pelvic pain, Transcutaneous Electrical Nerve Stimulation (TENS) is an effective therapeutic option through electrostimulation equipment, which allows pain control without the need of drugs an in a noninvasive manner.

### DESCRIPTION OF THE PRIOR ART

The usual electrostimulation equipment can be divided into two large groups: the ones with a bench, powered by the electric network, and the portable ones, powered by batteries. For the use of bench equipment, it is necessary to move the user to the places where the device is available, said device being usually operated by specialized people (physician or physiotherapists). On the contrary, the portable equipment is self-applicable and, after a professional has been indicated, the user can use it himself/herself in his/her home or work environment.

Among the already known portable electrostimulation devices specifically intended for pelvic pain control, let us mention the ones set forth in documents N°s. US 4.803.986 and US 2012/0109233 (corresponding to WO 2010/035962).

In document N°. US 4.803.986, the disk-shaped body of the eletrostimulator is equipped with an on-off button, a light indicator to warn if the device is switched on and/or if the battery is running out (requiring recharging), and with two control buttons, each one designed to increase/decrease the amplitude of the electric pulses to be supplied to each of the two output channels provided in the apparatus, respectively interconnected, through electrical connectors to the electrodes. The body of the electrostimulator is further provided with flaps which allow attachment of the apparatus to a strap or belt, attachable, for example, to the bra or around the waist of the user.

In document N°. US 2012/0109233, the electrostimulator comprises a cushion portion that includes a stimulation electrode, and a heating portion, which includes a planar heater, said electrostimulator thereby providing electrical and thermal stimulation. Such equipment is also powered by a large rechargeable battery, preferably lithium-ion battery.

The portable electrostimulation equipment currently in the market exhibits a great inconvenience: their dimensions are still considerable, due to the dimensions of the batteries used therein. In fact, the operations carried out by the electrical stimulation equipment require a large current consumption, which is the reason why the batteries used therein need to have sufficient capacity to meet this demand, and to power the equipment for a period that is considered acceptable. The batteries that meet these requirements show considerable sizes.

For this reason, the use of smaller, disposable batteries such as coin-shaped lithium-ion batteries has never been feasible, since these, having lower charge capacity, would substantially reduce the shelf life of the equipment.

Another disadvantage of the batteries commonly used in these portable equipment is the need to recharge them. In fact, it happens quite often a situation in which the equipment is discharged when the user is affected by the pain resulting from dysmenorrhea; in these cases, the user does not always have a place to recharge her/his device, and often, depending on the intensity of the pain, she/he cannot wait for this operation, and ends up resorting more readily to medicines. Ideally, the user should have a cheap and non-rechargeable electrostimulator equipment always ready for use, anytime, anywhere.

Furthermore, the portable electrostimulation equipment of the market has one more drawback: the need to be regulated by the user, through intensity control buttons provided in the body of the device. Since these devices are almost always applied in the suprapubic region or the lumbar region of the user, which are the preferential zones for the application of stimuli for the control of pain resulting from dysmenorrhea, the control buttons are difficult to visualize and access, making the activation thereof by the user difficult and cumbersome. This often turns out to be truly a hassle for the woman, who is already very fragile due to the pain, causing her to frequently abandon the use of electrical stimulation and to use the ready intake of medicines.

On the other hand, it is also known that to be effective in the treatment of pain, electrical stimuli (or pulses) must meet a series of requirements in terms of their intensity (or amplitude) [in volts (V)], their frequency [in hertz (Hz)], their width (or duration) [in microseconds (µs)], its waveform, and the used stimulation module.

In the scientific literature, there are several reports showing that analgesia induced by electrical pulses occurs within a very elastic range of the previously-mentioned parameters.

In fact, the numerous electrostimulation devices known in the art usually apply electrical pulses whose parameters are found in the following ranges:
- current intensity: 1-50 mA (charged at **500Ω**)
- frequency: 1-250 Hz;
- pulse-width (or duration): 10-1000 µs;
- waveform: single-phase, symmetrical biphasic, asymmetrical biphasic; and
- stimulation mode: continuous or intermittent.

Although there is extensive literature attesting to the efficacy of TENS, the mechanism of action is not fully understood, and the Theory of the Pain Portal and the Central Release of Endorphins are the most accepted mechanisms of action by the scientific community.

A well-known physiological phenomenon is the one of the nerve fiber accommodation at the electric stimulus. It is the refractoriness of the nerve cell membrane when the stimulus is applied in the same phase and with fixed parameters of intensity, frequency, and pulse width. In this case, the stimulation ceases to be effective and the analgesic effect does not occur.

To avoid the nerve fiber accommodation, several strategies have been developed, among them:
- inversion of the polarity of the electrical pulses;
- waveform change;
- variation of the frequency or intensity (amplitude) value of the electrical pulses.

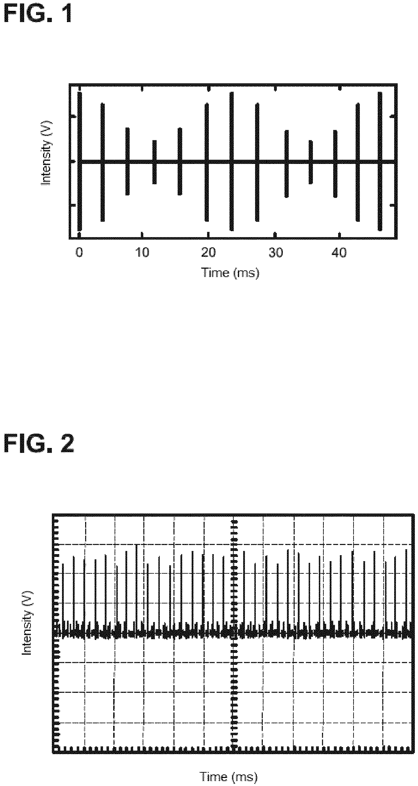

When the variation of the intensity (amplitude) of the electrical pulses is used as a strategy to prevent the accommodation of the nerve fibers, this variation is always done in a regular way in time (as shown in the graphical representation of attached Figure 1)

However, even when adopting this measure, there is still some degree of nerve fiber accommodation, precisely because there is a regular temporal repetition of said intensity variations. As a function of the plasticity and adaptive capacity of the cell membranes of the nerve fibers, regular intervals of variation allow said membranes to adapt, as well as cause the nerve fibers to be accommodated, thereby losing or reducing the analgesic effect.

This is another inconvenient of the electrostimulation equipment currently available in the market: the difficulty in maintaining nerve fiber non-accommodation when applying the electrical pulses.

Therefore, it would be desirable to develop some kind of protocol for the application of electrical stimuli that would be able to reduce nerve fiber accommodation in a more efficient way, and, therefore, to guarantee the prolonged analgesic effect.

On the other hand, it would also be desirable to obtain a portable equipment for electrostimulation application that consumes less current when applying electrical pulses, allowing the use of batteries of smaller size and capacity, resulting in a disposable cheaper and smaller equipment, guaranteeing better portability characteristics than the prior art equipment.

Moreover, it would be desirable to obtain a portable electrostimulation apparatus which would not require user's regulation, to make its use substantially easier than that of conventional equipment, which is difficult to operate.

### PURPOSES OF THE INVENTION

In order to achieve these purposes, an innovative electrostimulation protocol was developed, in which the intensity variation of the applied electrical pulses was randomly performed, respecting the limits stimulation efficacy. With this new protocol, it has been possible to effectively reduce the accommodation of cell membranes of nerve fibers, increasing electrostimulation efficacy and, thus, the analgesic effect.

Concurrently, this random variation of electrical pulse intensity has substantially reduced current consumption in the operations of the electrostimulation equipment. This feature allowed the use of disposable coin-shaped lithium-ion batteries, model CR20-XX, which have smaller sizes, lower cost, and lower charge capacity, but sufficient to meet the current lower current consumption, thanks to the intensity variations determined by the innovative electrostimulation protocol. Thereby, the disposability of the product has become feasible, since it is not necessary to replace the batteries.

Only this family of batteries combines the characteristics of reduced size, sufficient voltage and charge, low environmental impact, and reduced cost, which makes it possible to dispose of the equipment.

Thus, since it is now possible to use such disposable batteries, it has been possible to substantially reduce the equipment sizes, which were larger because they used to depend on batteries with greater capacity, which until then were necessary to provide the electrostimulation of the apparatus. Moreover, with the use of disposable batteries, it has become possible to significantly reduce the cost of the equipment.

Thus, with the creation of this new electrostimulation protocol, it has become possible to develop a low-cost, small- sized disposable equipment with great portability. Thereat, it is possible to use the equipment at any time and in any place, at home or at work, avoid unnecessary displacements, such as costs associated thereto.

On the other hand, the inventor(s) introduced significant changes in the electronics and mechanics of the equipment, in the sense of making it a one-touch actuating device, which substantially facilitated its use by users, who no longer need to use control bottoms to regulate the intensity of the electric pulses.

In fact, and unlike the conventional equipment, in this innovative equipment, the operation is done through a single on-off button, the activation of which initiates a sequence of automated electrostimulations, following this innovative electrostimulation protocol, after which the equipment is

automatically switched off. Hence, not only does it facilitate the use of the apparatus, but it also avoid, in a totally safe manner, the occurrence of errors resulting from the user's incorrect control of the control buttons.

### DESCRIPTION OF THE DRAWINGS

To complement the present description, to better understand the characteristics of the subject matter of the patent, a set of drawings accompanies this specification, in which, in an exemplified and nonlimiting manner, the following has been represented:
- Figure 1 is a graphical representation showing the usual strategy for avoiding the nerve fiber accommodation used in the known electrostimulation protocols, that is, the use of intensity (amplitude) variation of the electrical pulses, variation that, to this day, is done regularly over time;
- Figure 2 is another graphical representation, now illustrating the novel strategy to avoid nerve fiber accommodation, provided by this innovative electrostimulation protocol, that is, the use of a random variation of electrical pulse intensity (amplitude), during the application of pulse bursts;
- Figure 3 shows, also by means of a graphical representation, one of the embodiment of this innovative electrostimulation protocol, where pulse bursts with a determined duration and with sequentially inverted polarity are continuously applied;
- Figure 4 illustrates, similarly by means of a graphical representation, other embodiments of this innovative electrostimulation protocol, that is, the intermittent mode, according to which pulse bursts are applied with a determined duration, and with sequentially inverted polarity, although providing a time interval between said pulse bursts, with a determined duration.
- Figure 5 is another graphical representation showing the steps of this innovative electrostimulation protocol;
- Figures 6 and 7 show this innovative portable electrostimulation equipment for pelvic pain control, respectively by bottom and top perspectives;

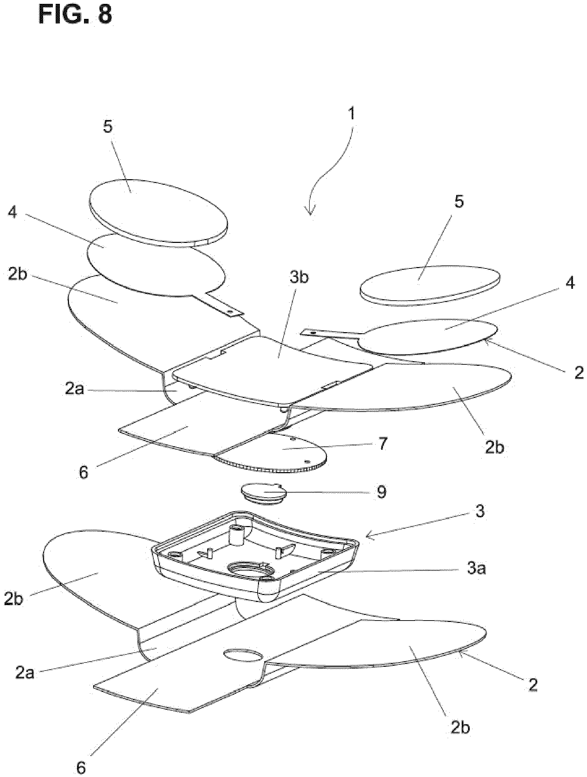

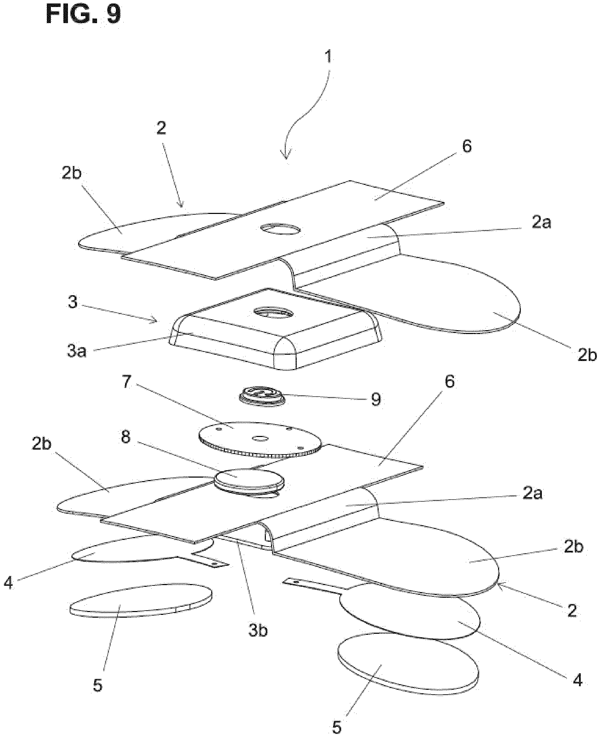

- Figures 8 and 9 show the same perspectives illustrated in Figures 5 and 6, now showing their components in exploded view;

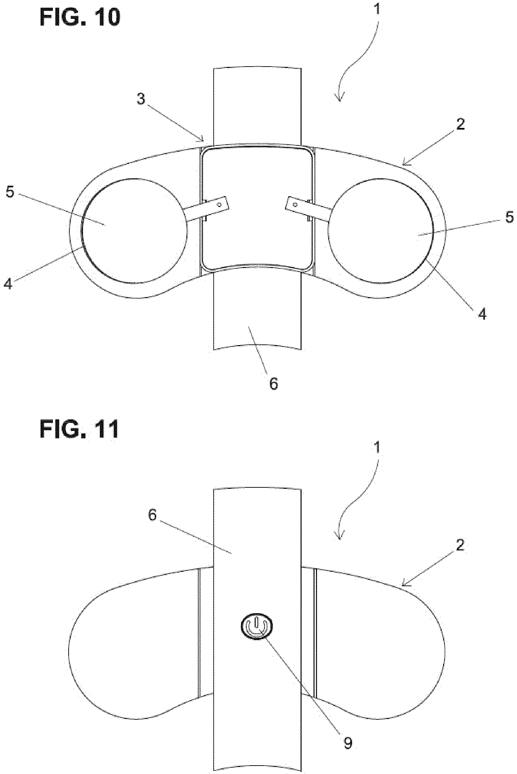

- Figure 10 is a bottom view of said equipment;
- Figure 11 is a top view of said equipment;

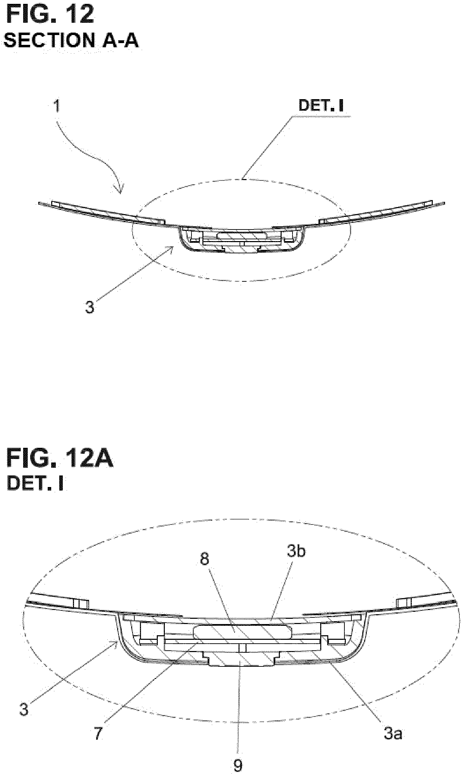

- Figure 12 is a section of the equipment, indicated by line A-A in Figure 11, wherein Figure 12A is an enlarged detail thereof, indicated in the previous figure;
- Figure 13 is another section of the equipment, indicated by line B-B in Figure 11, wherein Figure 13A, is an enlarged detail thereof, indicated in the previous figure;
- Figure 14 is a block diagram of said equipment;
- Figure 15 is the electric scheme of this innovative equipment;
- finally, Figure 16 is a flowchart of the software, specifically developed for this innovative electrostimulation protocol.

### DETAILED DESCRIPTION OF THE INVENTION

The present patent relates to a "PROCESS FOR ESTABLISHING AN ELECTROSTIMULATION PROTOCOL FOR PELVIC PAIN CONTROL, AND THE RESPECTIVE PORTABLE ELECTROSTIMULATION EQUIPMENT FOR PELVIC PAIN CONTROL USING SAID PROTOCOL", said electrochemical process and equipment being more specifically used for controlling the pain deriving from dysmenorrhea, endometriosis and other painful conditions of women's pelvic cavity.

With references to the "PROCESS FOR ESTABILISHING AN ELECTROSTIMULATION PROTOCOL", said process provides the development of an electrostimulation protocol in which intensity variation of the electrical pulses is performed in a random manner, respecting the limits of stimulation efficacy, to reduce the physiological phenomenon of nervous fiber accommodation upon receiving stimuli.

More specifically, according to the present process, the electrical pulses have a square wave shape, are monopolar, have a width (or duration) between 60 µs and 100 µs, preferably 80 µs, and a frequency between 40 Hz and 70 Hz, preferably 55 Hz, and Intensity varying randomly within a range of 10 V, preferably 5 V, as shown in attached Figure 2.

Furthermore, the present process discloses two embodiments for this innovative electrostimulation protocol, namely, continuous mode and intermittent mode.

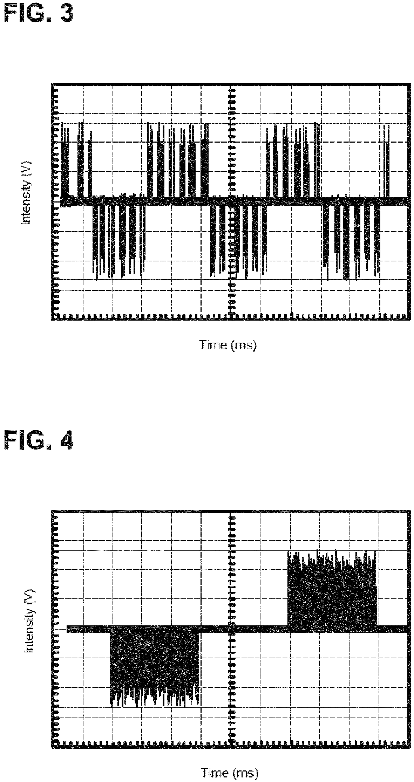

In the continuous mode, shown in the graphical representation of Figure 3, burst trains lasting 500 milliseconds to 2 seconds, preferably 1 second, and with sequentially inverted polarity are continuously applied.

In the intermittent mode, shown in the graphical representation of Figure 4, pulse bursts lasting from 1 to 4 seconds, preferably 3 seconds, and with sequentially inverted polarity are applied, however there is a time interval from 1 to 4 seconds, preferably 3 seconds.

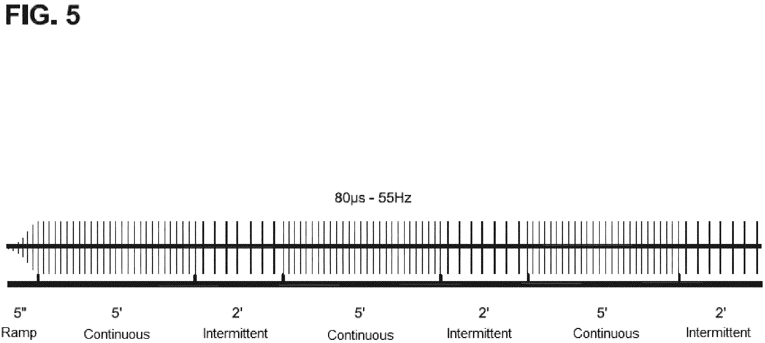

Due to this random variation of electrical pulse intensity throughout the stimulation cycle, the novel electrostimulation protocol for the control of pelvic pain presents the following steps, as shown in attached Figure 5:
a) intensity ramp from 0 V to 25 V peak [measured with resistive charge of 500Ω], for 5 seconds;
b) 1^{st} period of continuous mode, maintained for 5 minutes;
c) 1^{st} period of intermittent mode, maintained for 2 minutes;
d) 2^{nd} period of continuous mode, maintained for 5 minutes;
e) 2^{nd} period of intermittent mode, maintained for 2 minutes;
f) 3 ^{rd} period of continuous mode, maintained for 5 minutes;
g) 3^{rd} period of intermittent mode, maintained for 2 minutes;
h) end of the protocol, after 21 minutes and 5 seconds.

In optional embodiments, this innovative protocol discloses the simultaneous use of other strategies used together with the random intensity pulse variation, as a complementary way to further avoid nerve fiber accommodation, and to make current consumption even smaller.

Among the strategies simultaneously employed with the aleatory (or random) variation of the pulse intensity, let us cite the following ones:
- use of monopolar pulse bursts, which allow saving battery charge;
- polarity inversion of the pulse bursts, avoiding muscular fiber accommodation effect;
- inclusion of intermittent modes of interleaved stimulation for the maintenance of analgesia, once the desired effect has been achieved.

By means of this new protocol, in which the intensity of the electric pulses has begun to vary in a random way, it was possible to efficiently reduce nerve fiber accommodation, increasing the effectiveness of the electrostimulation and, thus, of the analgesic effect.

Concurrently, this random variation of electric pulse intensity further allowed a substantial reduction in current consumption in the operations of the electrostimulation equipment, allowing the use of standard coin-shaped lithium ion batteries, model CR20XX, which are smaller, cheaper and of lower load capacity, but sufficient to meet the current lower current consumption due to random intensity variations, determined by this innovative electric stimulation protocol, without the need to replace them.

Thus, it is now possible to use such common ion-lithium batteries, substantially reducing the dimensions of the equipment, which were greater because of the need of higher capacity batteries to provide electrostimulation operations of the apparatus.

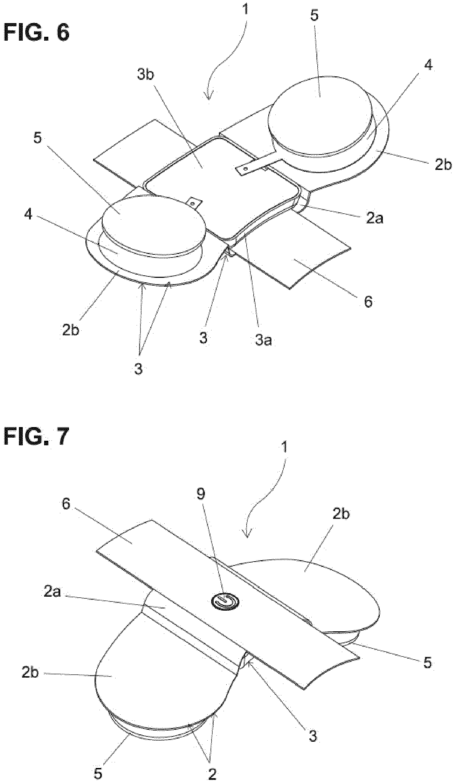

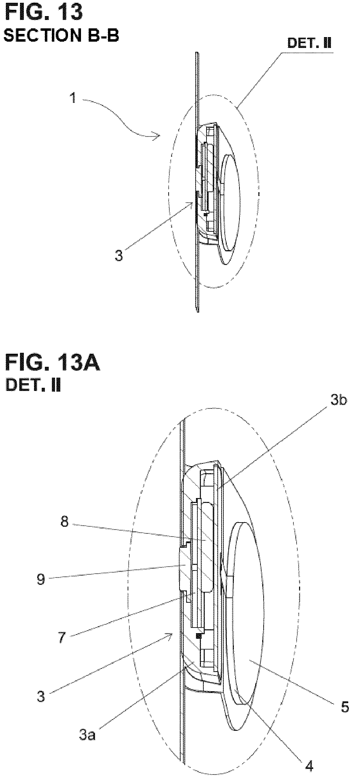

Regarding this "PORTABLE ELECTROSTIMULATION EQUIPMENT FOR PELVIC PAIN CONTROL USING SAID PROTOCOL, said equipment is shown in details in attached Figures 6 and 13.

Such equipment is constituted by a double bandage (1) formed by two sheets (2), each comprised of a central portion (2a) and two side flaps (2b), the central portion (2a) of both sheets (2) housing between them a compartment (3) formed by a body (3a) and a cover (3b), which forms a housing for the electronic module of the apparatus and the two side flaps (2a) of the top sheet (2) housing the respective electrodes (4), duly covered by the respective layers of gel protected by the removable protective sheets (5); each of the sheets (2) is additionally endowed with a respective fixing strip (6), which joins the sheets to the electronics compartment of the equipment.

As already mentioned, the central compartment (3) houses the electronic module of the apparatus, consisting of a printed circuit board (7) with the electrical components and circuit of the apparatus; said central compartment (3) also houses the power supply battery (8), which, thanks to the innovative electrostimulation protocol hereinbefore provided, could now be a rechargeable coin-shaped ion-lithium battery, model CR20XX.

This innovative equipment discloses a single on-off button (9), which, upon actuation, starts the above-described electrostimulation protocol, automatically switching off the device, at the end of said protocol.

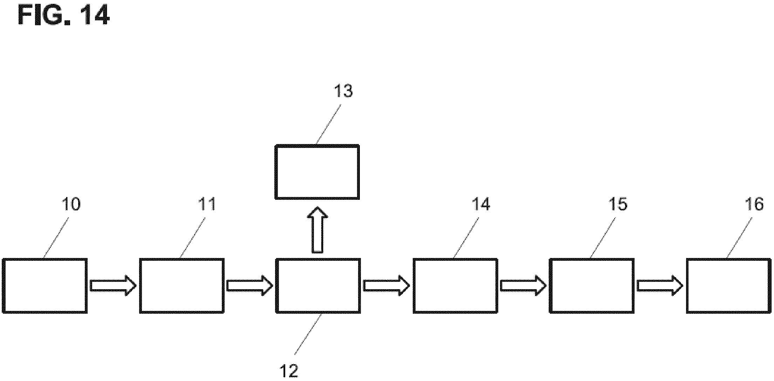

The block diagram of Figure 14 illustrates the internal components of the electronic module of this innovative equipment, namely: Power source module (10), step-up regulator module (11), micro controller module (12), power supply seal module (13), boost source module (14), H-bridge module (15), and electrode output module (16).

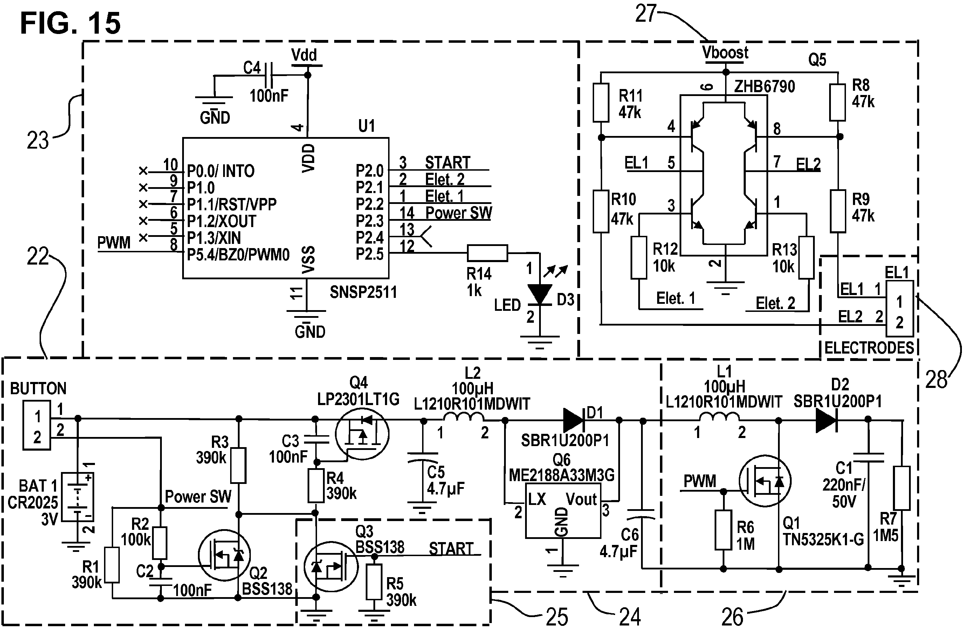

These modules will be explained in details below, together with the description of this innovative electrical equipment, shown in attached Figure 15.

As verified in said Figure 15, circuit (17), which refers to the Power Supply Module (10), supplies all the other circuits with the voltage supplied by the battery. When MOSFET "Q2" is saturated with the signal coming from the power bottom, it will saturate MOSFET "Q4", energizing the circuit (19), which refers to the Step-up Regulator Module (11).

Circuit (19) has a "Q6" Step-Up Regulator Module, which acquires the voltage of the battery and raises it to 3.3 V. This output voltage is filtered by the capacitor "C6", and then supplied to the circuit (21) and to power the microcontroller.

In the circuit (18), which refers to the Microcontroller Module (12), there is microcontroller "U1", which is responsible for controlling all functions of the equipment. Once it has been powered, the "D3" LED flashes in an intermittent mode to indicate the operation of the equipment. Furthermore, the microcontroller then monitors the power button signal, sends the PWM signal to "Q1", sends the signals to control the H-Bridge and sends a signal to "Q3" in the circuit (20), which refers to the Power Seal Module (13), responsible for keeping the MOSFET "Q4" of the circuit (17) saturated, which makes it unnecessary for the Power button to be pressed to allow the equipment to remain energized.

Circuit (21), which refers to Boost Source Module (14) is a boost-type DC/DC converter whose function is to raise the voltage range of 3.3 V to the level that will be used in electrostimulation. The converter is controlled by a PWM signal applied to the MOSFET "Q1 ", and the output voltage is sent to the circuit (22) through the capacitor "C1 ".

Circuit (22), which refers to a H-Bridge Module (15), has the function of discharging in the electrodes the energy stored in the capacitor "C1" of the boost source. Another feature is to reverse the polarity of current flowing through these electrodes, said inversion occurring according to the digital signals emitted by the microcontroller. The width of the pulses outcoming from the H-bridge is also controlled by the same signal which controls polarity reversal, and then, finally, these stimuli are conducted to the user through the electrodes represented by the block (23) in Figure 15, which refers to the Electrode Output Module (16) in the block diagram of Figure 14.

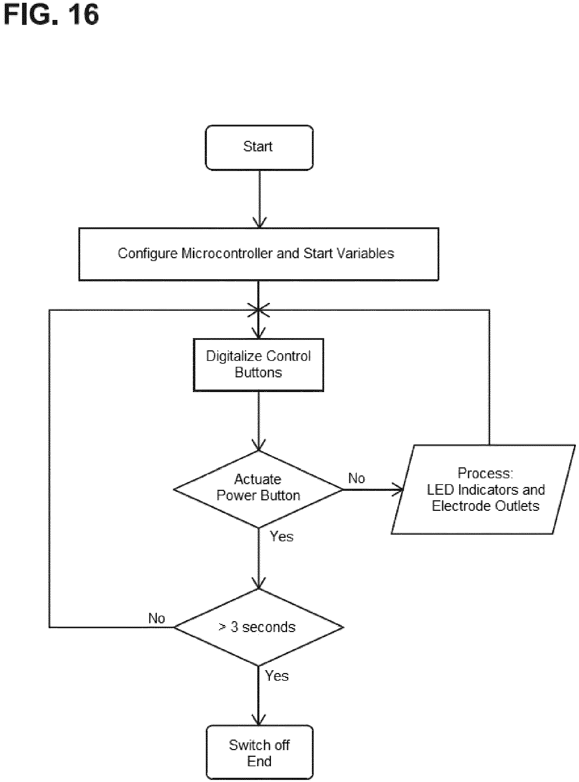

Figure 16 is a flowchart of the software specifically developed for this innovative electrostimulation protocol for this now innovative pelvic control electrostimulation protocol.

As previously mentioned, not only does the new protocol of electrostimulation created by the Inventor(s) effectively reduce nerve fiber accommodation, but it makes also possible to reduce current consumption in the operations of the electrostimulation equipment, allowing the use of a disposable coin-shaped lithium-ion battery (8), model CR20XX, with smaller sizes, low cost and lower charge capacity, but sufficient to meet the current lower current consumption thanks to the random intensity variations determined by the innovative electric stimulation protocol.

Hence, it has become possible to develop the innovative electric stimulation equipment, which is disposable, cheap, compact and portable, making it possible for users to use it at any time and in any place, at home or at work.

Moreover, thanks to the alterations introduced by the Filing Applicant in the electronics and mechanics of the equipment, it was possible to make it a one-touch actuating device, which substantially facilitated its use by users, who no longer need to use control bottoms to regulate the intensity of the electric pulses.

In fact, in this innovative equipment, the operation is carried out through a single on-off button (9), whose activation initiates the automated electrostimulation sequence, following this innovative electrostimulation protocol, after which the equipment is automatically switched off. Hence, not only does it facilitate the use of the apparatus, but it also avoid, in a totally safe manner, the occurrence of errors resulting from the user's incorrect control of the control buttons.

## Claims

1. A PROCESS FOR ESTABLISHING AN ELECTROSTIMULATION PROTOCOL FOR PELVIC PAIN CONTROL **characterized in that** it provides a random variation of electrical pulse intensity, respecting the stimulation efficacy limits, wherein said random variation is comprised in the range of 10 V, and said electrical pulses exhibit a square wave shape, monopolar polarity, a width (or duration) between 60 µs and 100 µs, and a frequency between 40 Hz and 70 Hz; and said electrostimulation protocol further involves the following steps:
a) intensity ramp from 0 V to 25 V peak [measured with resistive charge of 500Ω], for 5 seconds;
*b) 1^{st} period of continuous mode, maintained for 5 minutes;*
*c) 1^{st} period of intermittent mode, maintained for 2 minutes;*
*d) 2^{nd} period of continuous mode, maintained for 5 minutes;*
*e) 2^{nd} period of intermittent mode, maintained for 2 minutes;*
*f) 3^{rd} period of continuous mode, maintained for 5 minutes;*
*g) 3^{rd} period of intermittent mode, maintained for 2 minutes;*
*h) end of the protocol, after 21 minutes and* 5 *seconds.*

2. The PROCESS FOR ESTABLISHING AN ELECTROSTIMULATION PROTOCOL FOR PELVIC PAIN CONTROL, according to claim 1, **characterized in that** electrical pulse intensity varies randomly preferably within the range of 5V.

3. The PROCESS FOR ESTABLISHING AN ELECTROSTIMULATION PROTOCOL FOR PELVIC PAIN CONTROL, according to claim 1, **characterized in that**
the width (or duration) of the electrical pulses is 80 µs, and/or that
the frequency of the electrical pulses is 55 Hz.

4. The PROCESS FOR ESTABLISHING AN
ELECTROSTIMULATION PROTOCOL FOR PELVIC PAIN CONTROL, according to claim 1, **characterized in that** it provides two electrostimulation modes, namely, continuous mode and intermittent mode.

5. The PROCESS FOR ESTABLISHING AN
ELECTROSTIMULATION PROTOCOL FOR PELVIC PAIN CONTROL, according to claims 1 and 4, **characterized in that** pulse bursts are continuously applied, in a continuous mode, with duration from 500 milliseconds to 2 seconds, and with sequentially inverted polarity.

6. PROCESS FOR ESTABLISHING AN ELECTROSTIMULATION PROTOCOL FOR PELVIC PAIN CONTROL, according to claim 5, **characterized in that** said pulse bursts exhibit a duration of 1 second.

7. The PROCESS FOR ESTABLISHING AN
ELECTROSTIMULATION PROTOCOL FOR PELVIC PAIN CONTROL, according to claims 1 and 4, **characterized in that**, in the intermittent mode, pulse bursts lasting 1-4 seconds and with sequentially inverted polarity, with a time intervals between the pulse bursts, are applied, wherein said interval lasts 1-4 seconds.

8. The PROCESS FOR ESTABLISHING AN
ELECTROSTIMULATION PROTOCOL FOR PELVIC PAIN CONTROL, according to claim 7, **characterized in that** said pulse bursts exhibit a duration of 3 second.

9. The PROCESS FOR ESTABLISHING AN ELECTROSTIMULATION PROTOCOL FOR PELVIC PAIN CONTROL, according to claim 7, **characterized in that** said time interval between the pulse bursts exhibits a duration of 3 second.

10. The PROCESS FOR ESTABLISHING AN ELECTROSTIMULATION PROTOCOL FOR PELVIC PAIN CONTROL, according to claim 1, **characterized in that** it optionally discloses the simultaneous use of other strategies both to avoid nerve fiber accommodation, used together with the random variation of pulse intensity.

11. The PROCESS FOR ESTABLISHING AN ELECTROSTIMULATION PROTOCOL FOR PELVIC PAIN CONTROL, accord
ing to claims 1 and 10, **characterized in that**
one of said strategy is the use of monopolar pulse bursts, that
the other of said strategies is pulse burst polarity inversion, and/or that
one of said strategy is the addition of intermittent stimulation modes.

12. A PORTABLE ELECTROSTIMULATION EQUIPMENT FOR PELVIC PAIN CONTROL USING SAID PROTOCOL, **characterized in that** it consists of a double bandage (1) of two sheets (2), each comprising a central portion (2a) and two lateral flaps (2b), the central portion (2a) of both sheets (2) housing between them a compartment (3) formed by a body (3a) and a cover (3b), which configures a housing for the electronic module of the apparatus, and the two side flaps (2a) of the top sheet (2) housing the respective electrodes (4), duly covered by the respective gel layers, protected by removable protective sheets; each one of the sheets (2) is additionally endowed with a respective fastening strip (6); the electronic module consists of a printed circuit board (7) with the electrical components and circuit of the apparatus, a disposable coin-shaped lithium-ion supply battery (8), model CR20XX and a single connecting button (9), whose activation initiates the electrical stimulation protocol, at the end of which the apparatus is automatically switched off, are provided; The following internal components are also provided: Power source module (10), step-up regulator module (11), micro controller module (12), power supply seal module (13), boost source module (14), H-bridge module (15), electrode output module (16).

13. The PORTABLE ELECTROSTIMULATION EQUIPMENT FOR PELVIC PAIN CONTROL USING SAID PROTOCOL, according to claim 12, **characterized in that** said disposable battery (8) is a 3V one.

14. The PORTABLE ELECTROSTIMULATION EQUIPMENT FOR PELVIC PAIN CONTROL USING SAID PROTOCOL, according to claim 12, **characterized in that** it comprises the electrical scheme shown in Figure 12.

15. The PORTABLE ELECTROSTIMULATION EQUIPMENT FOR PELVIC PAIN CONTROL USING SAID PROTOCOL, according to claim 12, **characterized in that** it comprises the flowchart shown in Figure 16.
